# EUROPEAN PATENT APPLICATION

(11) **EP 4 714 492 A1**
(43) Date of publication of application: **25.03.2026**
(21) Application number: 24806722.5
(22) Date of filing: 15.05.2024
(51) Int. Cl.: A61N 5/10

(54) **POSITIONING AND LOCALISATION DEVICE FOR EXTERNAL RADIATION THERAPY ON GYNAECOLOGICAL TUMOURS**

(30) Priority: 16.05.2023 ES 202330377
(71) Applicant: Servicio Andaluz de Salud, 41071 Sevilla (ES)
(72) Inventor: VELÁZQUEZ MIRANDA, Santiago, 41013 Sevilla (Sevilla) (ES)
(74) Representative: San Martin Alarcia, Esther
(86) International application number: PCT/ES2024/070291
(87) International publication number: WO 2024/236211

(57) **Abstract**

The invention discloses a localisation device (1) for radiation therapy on gynecological tumours that comprises: a frame (2) comprising a longitudinal guide (21), where said frame (2) is configured to be attached to an immobiliser for radiation therapy; a trolley (3) longitudinally and slidingly attached to the longitudinal guide (21) of the frame (2); and a bar (4) removably and slidingly attached to the trolley (3) in a direction parallel to the longitudinal guide (21). The bar (4) is placed a suitable distance from the frame (2) for its insertion into the vagina of a patient who is located on the immobiliser for radiation therapy in the supine position and with bent legs during radiation therapy treatment or medical imaging. Furthermore, at least the bar is made with a material having an electron density that is essentially the same as that of water.

## Description

### OBJET OF THE INVENTION

The present invention falls within the field of medicine, and more specifically within the field of treatment of gynaecological tumours.

The object of the present invention is a new device designed to accurately position and locate a tumour located in the uterus or vagina. This allows external radiotherapy treatments to be performed in cases where only brachytherapy is currently applicable.

### BACKGROUND OF THE INVENTION

There are different techniques for the treatment of cancer, among which external radiotherapy and brachytherapy are well known. External radiotherapy consists of attacking the tumour with external beams of high-energy radiation from linear electron accelerators. Brachytherapy consists of the introduction of a radioactive source into the patient's body, next to the tumour.

External radiotherapy has many advantages over brachytherapy. These advantages range from the elimination of the danger of handling radioactive sources, to a significant reduction of costs, the ability to treat lesions that are too large for brachytherapy, or the improvement of the treatment experience.

However, despite its advantages, external radiotherapy is not applicable to certain types of gynaecological cancers. The main reason is that the vagina and/or uterus, where the treatment target is located, are highly mobile organs whose shape and position can vary in seconds without synchronised correlation with breathing. This prevents accurately locating the tumour following the usual treatment consisting of, first, locating the tumour using a computed tomography (CT) or magnetic resonance (MR) apparatus, and subsequently performing the treatment by the linear accelerator (LINAC) at the previously determined location. The position of the tumour determined by CT or MR would not correspond to the position at the time of treatment in the LINAC. Nor does the use of CT normally integrated into LINACs provide a solution, as these are low-resolution CTs that do not allow adequate localisation of the treatment target.

For these reasons, the treatment currently used for this type of gynaecological tumour is brachytherapy.

### DESCRIPTION OF THE INVENTION

The inventor of the present invention has developed a positioning and localisation device that makes it possible to locate with millimetre precision the position of the tumour to be treated, thus enabling radiotherapy to be applied to gynaecological tumours that, until now, were only treatable by means of brachytherapy.

The main concept of the device is the arrangement of a bar configured for its insertion into the vagina of the patient, this bar having a series of marks visible by means of CT or MR and, above all, being made of a material with an electron density similar to that of water. The bar is inserted into the patient's vagina in a reproducible position during a first step of locating the tumour using a CT or MR machine. By having water density, this bar does not interfere with the imaging processes of the CT or MR machine, allowing the tumour to be located with precision. Subsequently, once the patient is positioned in the LINAC in the same position as in the CT or MR machine, the bar is reinserted until reaching the reproducible position mentioned above, which allows knowing with millimetre precision the location of the tumour.

In contrast, brachytherapy devices do not have water density and even have metal components that prevent dose calculation with current commercial planners. The invention, with its new aqueous density, not only does not produce artefacts in the images like usual brachytherapy devices, but allows for a calculation and distribution of the correct radiation dose. The removal of the transport mechanisms from radioactive sources allows this change of materials in the uterine and vaginal positioners.

In this document, the term *"radiotherapy immobiliser*" refers to a device designed to immobilise a patient during a radiotherapy treatment. Such an immobiliser usually comprises a base, normally in the form of an essentially flat board, which supports the patient and to which are attached a series of elements that constrain the patient to reduce their movements as much as possible. Document US2012167898A1 is an example of a radiotherapy immobiliser of this type. In this context, the main direction of a radiotherapy immobiliser is a direction parallel to the craniocaudal direction of a patient when the patient is in a supine position on the immobiliser in the natural position of use.

In this document, positional terms such as "vertical," "up," "down," and the like are interpreted assuming that the device of the invention is attached to a radiotherapy immobiliser and that the immobiliser is in its natural position of use supported on the accelerator table, unless the context clearly indicates otherwise.

In this document, the terms "proximal" and "distal" are interpreted as referring to the position of the medical professional who is operating the device, unless the context clearly indicates otherwise. That is, those elements closest to the medical professional are referred to as "proximal," and those elements farthest from the medical professional will be referred to as *"distal."*

The locating device of the present invention fundamentally comprises the following elements: frame, trolley and bar. These elements are described in more detail below.

### a) Frame

It is an essentially flat frame comprising a longitudinal guide, said frame being configured for slidingly attached to a radiotherapy immobiliser in such a way that the longitudinal guide is oriented parallel to a main direction of said radiotherapy immobiliser.

Normally, the frame will be formed by a flat plate that rests on the immobiliser and moves resting on it with the aid of means for attaching to the immobiliser. Thus, when the frame is attached to the immobiliser, normally to an end of the immobiliser on which the patient's feet rest during use, the longitudinal guide is parallel to the main direction of the immobiliser and, therefore, to the craniocaudal axis of the patient.

In principle, the frame may have any suitable configuration as long as it is attachable to the immobiliser in the manner mentioned above. In a particularly preferred embodiment of the invention, the frame comprises a square or rectangular frame with the guide arranged between two opposite sides and perpendicular thereto. The guide may have any configuration as long as it allows the trolley, which will be described later, to slide along it. In addition, the guide preferably comprises locating marks visible by means of computed tomography and magnetic resonance apparatus. These are stereotactic references that correlate the exterior of the patient visible to the naked eye with the internal references to the patient only visible by CT or MR. These stereotactic references will allow to accurately reproduce the position of the trolley according to the needs of each application.

Furthermore, the means of attaching the frame to the radiotherapy immobiliser can be configured in any manner known in the art so that the frame can slide along the immobiliser. Normally, it is sufficient for these means to allow the frame to slide perpendicular to the main direction of the immobiliser, although they could also be configured to allow sliding parallel to said main direction. Thus, when the immobiliser has a rail or similar, for example a rail perpendicular to the main direction located at that end of the immobiliser, the attaching means of the frame will be configured to attach the frame to said rail in a sliding manner.

### b) Trolley

The trolley is slidingly attached to the longitudinal guide of the frame so that it can move in both directions along said longitudinal guide.

In principle, the trolley could have any configuration as long as it can slide along the frame guide. Specifically, in a particularly preferred embodiment of the invention, the trolley comprises a base and a tower attached to the base in a removable manner. The base is slidingly attached to the frame guide, while the bar is attached to the tower in a removable and sliding manner. Even more preferably, the base comprises a prismatic cavity with an axis perpendicular to the plane of the frame, inside which a portion of the tower opposite to that to which the bar is attached fits.

This configuration allows for a multiplicity of adjustments and movements to adapt the device to each specific patient. Firstly, it is possible to have several towers of different dimensions to use the most appropriate one in each case. Secondly, the bar can also be disassembled to use that having the most suitable dimensions. In addition, the slidability of the base along the frame guide allows the tower to be moved closer to the position of the patient's vagina, and subsequently, the ability to slide the bar relative to the tower allows the bar to be inserted into the patient's vagina. The procedure for using this device will be described in greater detail later in this document.

### c) Bar

The bar is attached to the trolley in a removable and sliding manner in a direction parallel to the longitudinal guide. In addition, the bar will be located at a distance from the frame that is suitable for insertion into the vagina of a patient lying in the radiotherapy immobiliser in supine position with bent legs during radiotherapy treatment or medical imaging.

In principle, the bar can be configured in any way as long as it can be inserted into the patient's vagina to a sufficient distance, in any case no greater than the natural depth of the vagina. For example, it may have an approximate length of about 40 cm, so that the part of the bar protruding from the trolley or, where applicable, from the tower attached to the base, is between 20 cm and 30 cm. As regards the diameter of the bar, it may be between approximately 1 cm and approximately 4 cm.

On the other hand, the distance between the bar and the frame may vary depending on the anatomy of each patient, although it is preferably between 10 cm and 25 cm.

The device may comprise a single bar slidingly attached to the trolley or, where applicable, to the tower. However, in a particularly preferred embodiment of the invention, the device further comprises an auxiliary bar detachably attached to the trolley parallel to the bar. This auxiliary bar will have different dimensions from those of the main bar, thus allowing the main bar to be replaced by the auxiliary bar quickly and easily when necessary. For example, the auxiliary bar may have a significantly smaller diameter than the main bar.

The sliding mechanism between the bars and the trolley, or where applicable the tower, may be any type as long as they slide parallel to the direction of the frame guide. When the device is attached to an immobiliser, this direction will coincide with the main direction of said immobiliser and will therefore allow the bar to be moved in a craniocaudal direction for insertion into the patient's vagina. For example, in a particularly preferred embodiment of the invention, the bars comprise two longitudinal slots located on diametrically opposite sides that are configured to slide into protrusions of a cavity in the trolley.

In another preferred embodiment of the invention, the bars comprise locating marks visible by means of computed tomography and magnetic resonance apparatus. These marks will allow the position of the tumour to be accurately located once the bar is inserted into the patient's vagina. The locating marks may take any suitable form, although they preferably comprise equispaced holes along the longitudinal direction of the bars.

In addition, the bar, and preferably also the frame and the trolley, is made of a material with an electron density essentially equal to that of water. The definition of the term *"electron density"* is well known in the field of medical physics. By way of example only, a complete definition of this concept can be found on the Wikipedia website: https://en.wikipedia.org/wiki/Electron_density.

As the bar is made of a material with an electron density equal to that of water, the device of the invention can be used in magnetic resonance or computed tomography machines to locate the tumour accurately before starting treatment in the linear accelerator. It also allows the use of the less accurate computed tomography scan function that linear accelerators usually have for the purpose of checking whether the tumour is in the position determined during planning. More specifically, as there is not enough contrast to distinguish between the tissues involved in the CT image of the LINAC, and the equipment is clearly distinguishable, the latter is taken as a reference to correct the millimetric position of the lesion with respect to the radiation beams, since it is possible to correlate the position of the equipment with the vagina and uterus.

In a particularly preferred embodiment of the invention, the device further comprises a protrusion disposed on a proximal portion of the frame for supporting an immobilisation mattress. In this context, an immobilisation mattress is a sort of cushion made of a hardenable material that is used to better fix the position of the patient. This protrusion may have any shape as long as it allows the immobilisation mattress to be fixed to the proximal portion of the frame for the purpose of providing support for the patient's legs. For example, the protrusion in this example has an essentially trapezoidal shape that protrudes vertically from the rear plate of the frame that forms the frame.

Another preferred embodiment of the invention is directed to a localization system for radiotherapy in gynaecological tumours comprising the following elements:
a) A locating device wherein the trolley is formed by a base and a tower as described in the preceding paragraphs.
b) A plurality of replacement towers for attaching to the base of the trolley, where each tower is configured for attaching the bar at a different distance from the frame.
c) A plurality of replacement bars of different diameters configured for attaching to the towers.

This system will allow the device of the invention to be adapted to the anatomy of different patients in a simple, quick and safe manner. In this context, it is important to note that these types of devices suffer significant wear due to their intensive use, and that more complex mechanisms for positioning the bar at the desired height could suffer breakdowns with excessive frequency. The system of the invention solves this problem thanks to its greater robustness.

### BRIEF DESCRIPTION OF THE DRAWINGS

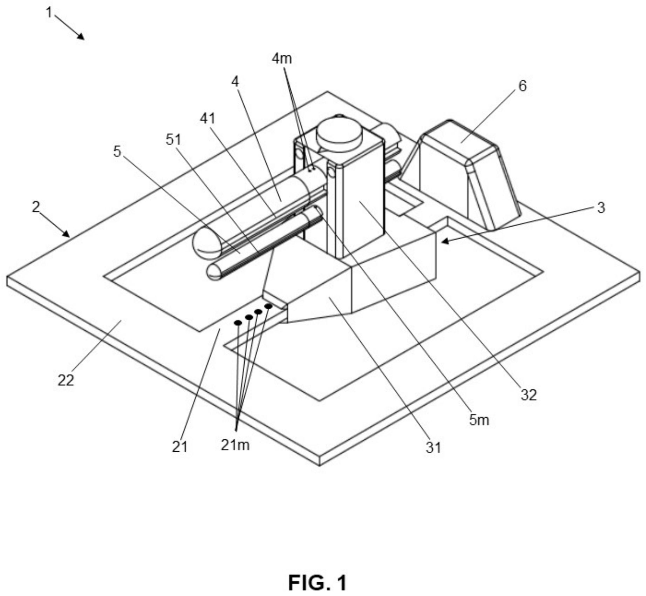

Fig. 1 shows a perspective view of an example of a device according to the present invention.
Fig. 2 shows a top view of the example of device of the invention shown in Fig. 1.
Fig. 3 shows a front view of the example of device of the invention shown in Fig. 1.
Fig. 4 shows a side view of the example of device of the invention shown in Fig. 1.

### PREFERRED EMBODIMENT OF THE INVENTION

A particular example of a device (1) according to the present invention is described below, showing in detail the different elements that compose it.

As can be seen, the device (1) is essentially formed by a frame (2) to which a trolley (3) is slidingly attached, to which a bar (4) is also slidingly attached.

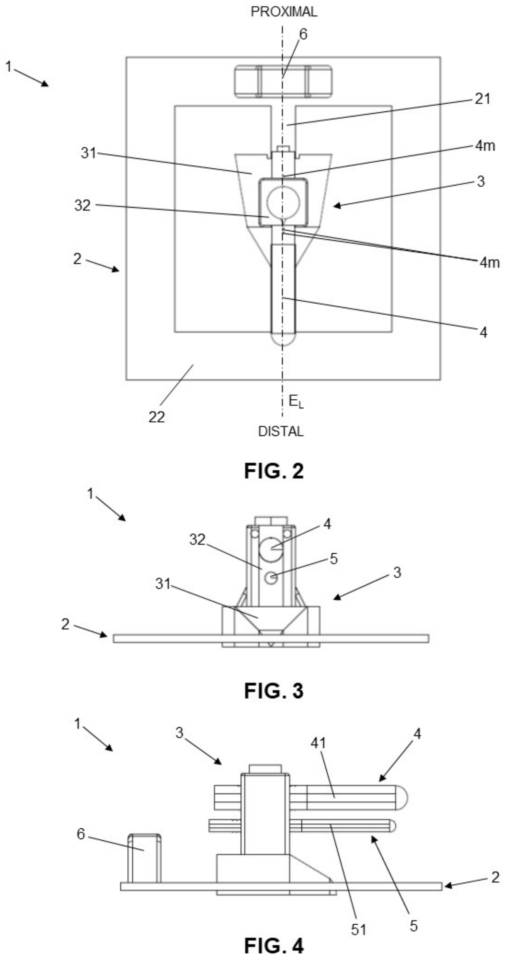

The frame (2) is a flat plate having an essentially square outer frame (22) which, according to the position shown in Fig. 2, comprises two lateral sides and two respectively upper and lower sides. A guide (21) is connected between the upper and lower side along a longitudinal axis (E_{L}) of the frame (2). In this example, the guide (21) is a part of the frame plate (2) itself, rectangular in shape and connected between the upper side and the lower side, although, as described above, it could have other alternative shapes. The guide (21) will have locating marks (21m) visible by MR or CT apparatuses, such as slots equispaced along its entire length to facilitate precise positioning of the trolley (3) during use of the device (1).

The trolley (3) shown in this example is formed by two separate pieces, a base (31) and a tower (32). The base (31) is configured for sliding attachment to the guide (21) of the frame (2). To that end, the base (31) has a longitudinal channel open at the bottom into which the guide (21) fits, so that said guide (21) acts as a rail on which the base (31) slides distally or proximally along the longitudinal direction. Although not shown in the figures, the base (31) and/or the guide (21) will have locking means that allow the base (31) to be fixed in a desired position.

The tower (32) also has a square prism shape, such that its lower portion fits inside a cavity in the base (31) that has a complementary shape. Specifically, it is a square prism-shaped cavity that is open at the top, i.e. open in a direction perpendicular to the plane of the frame (2). Although not shown in the figures, these elements may have connecting means configured to fix the tower (32) inside the cavity of the base (31).

Furthermore, although not shown in the figures, the tower (32) may have vertical slots located on its side faces, i.e., the faces perpendicular to the longitudinal direction. The base cavity (31) can have complementary protrusions also located on its side faces. The protrusions of the cavity in the base (31) will enter the slots in the tower (32) to facilitate the guidance of the tower (32) during the attaching process between the two components.

The tower (32) also has a longitudinal hole configured to receive a main bar (4) in a longitudinally sliding manner. The main bar (4) has an essentially cylindrical shape with a rounded tip, although other shapes that facilitate insertion into the patient's vagina would also be possible. In this specific example, the length of the main bar (4) is approximately 40 cm, and its diameter is 4 cm.

The mechanism that allows the main bar (4) to slide in relation to the tower (32) can be any, although in this example the main bar (4) has two longitudinal slots (41) arranged in diametrically opposite positions which, in the attaching position, are in a plane parallel to the frame (2). In turn, on the two side walls of the interior of the longitudinal hole of the tower (32) there are ribs that are also parallel to the frame (2). In this way, the bar (4) can slide in the proximal or distal direction along the longitudinal direction. Although not shown in these figures, the bar (4) and/or the tower (32) has locking means configured to fix the bar (4) in a desired position.

The device (1) in this example also comprises an auxiliary bar (5) arranged parallel to the main bar (4). To that end, the tower (32) comprises an auxiliary hole also oriented longitudinally and located closer to the plane of the frame (2). The attaching of the auxiliary bar (5) to the auxiliary hole in the tower (32) is the same as that described in relation to the main bar (4). Thus, the auxiliary bar (5) also has two longitudinal slots (51) arranged in diametrically opposite positions which, in the attaching position, are in a plane parallel to the frame (2). In turn, on the two side walls of the interior of the auxiliary hole in the tower (32) there are ribs also parallel to the frame (2). In this way, the auxiliary bar (5) can slide in the proximal or distal direction along the longitudinal direction. Although not shown in these figures, the auxiliary bar (5) and/or the tower (32) has locking means configured to fix the auxiliary bar (5) in a desired position.

In addition, the main bar (4) and the auxiliary bar (5) both comprise locating marks (4m, 5m) visible by MR or CT apparatus. These marks (4m, 5m) may be through holes that pass through said bars (4, 5) at equal intervals in a direction perpendicular to the plane of the frame (2). The locating marks will allow the main bar (4) or the auxiliary bar (5) to be placed in a precise, repeatable position during use of the device (1).

The device (1) in this example also comprises a protrusion (6) fixed to the proximal side of the frame (22) of the frame (2) that protrudes perpendicular to the plane of said frame (2). This protrusion (6) provides support for an immobilisation mattress.

Finally, it is important to note that the frame (2), the trolley (3) and the bar (4) are made of a material with an electron density similar to that of water, i.e. a value of 1. For example, these components may be made of a plastic material of the type commonly used in 3D printing machines. This ensures that the device (1) as a whole is fully compatible with the CT or MR machines normally used for the precise localisation of the tumour in this type of procedure.

Thus, the use of this device (1) for radiotherapy treatment of a gynaecological tumour would be as follows.

First, a tumour localisation step is performed. To do this, the immobiliser is placed on the table of an MR or CT apparatus. The patient lies on the immobiliser in a supine position with the legs bent. The device (1) of the invention is slidingly attached to the end of the immobiliser where the patient's legs are located. The medical professional moves the device (1) at least in a direction perpendicular to the main direction of the immobiliser until the bar (4) is placed in a centered position relative to the patient. The device (1) of the invention is fixed in this position. A tower (32) whose hole is at a height above the frame (2) appropriate to the position of the particular patient's vagina is selected. A bar (4) of a length and diameter appropriate to the anatomy of the patient's vagina is also selected. The bar (4) is fixed to the main hole of the tower (32) in a rear position, i.e. by sliding the bar (4) in the proximal direction to leave space on the distal side of the tower (32). The auxiliary bar (5) is removed from the tower (32) so that it does not interfere during the procedure. The trolley (3) is advanced along the longitudinal guide (21) to the most distal position possible. This position is usually limited by the patient's own thighs, so it is possible that the bar (4) has not yet reached the entrance to the vagina. The bar (4) is slid longitudinally in the distal direction along the hole in the tower (32) until it is inserted the patient's vagina, reaching a position where it rests against the wall of the vagina opposite the entrance. Next, the images that are necessary are taken, locating the position of the tumour precisely using the locating marks on the bar (4) as a reference. The locating marks on the bar (4) also allow determining the position of the bar (4) in relation to the tower (32). Since the device (1) is made of a material with unit electron density, it does not interfere in any way with the images acquired by CT or MR.

Secondly, a step of treating the tumour is carried out. To that end, the immobiliser is placed on the table of a linear accelerator. The patient lies down on the immobiliser in the same position as in the MR or CT apparatus, i.e. in a supine position with their legs bent. The radiotherapy immobiliser has elements that constrain the patient to adopt a position as similar as possible. Next, the device (1) of the invention is fixed to the immobiliser in the same position as it was fixed in the stage described above. The trolley (3) is moved to the same position as in the previous step, and the bar (4) is inserted into the patient's vagina, also to the same position. The locating marks are used to determine the position of the tumour. After performing accuracy checks using the low-precision CT of the linear accelerator, radiotherapy is applied according to the parameters established during planning.

## Claims

1. Positioning and localisation device (1) for radiotherapy in gynaecological tumours, **characterised in that** it comprises:
- an essentially flat frame (2) comprising a longitudinal guide (21), said frame (2) being configured for sliding attaching to a radiotherapy immobiliser such that the longitudinal guide (21) is oriented parallel to a main direction of said radiotherapy immobiliser;
- a trolley (3) slidingly attached to the longitudinal guide (21) of the frame (2) so that it can move in both directions along said longitudinal guide (21), wherein the trolley (3) comprises a base (31) and a tower (32) directly attached to the base (31) in a removable manner, the base (31) being directly attached in a sliding manner to the guide (21) of the frame (2); and
- a bar (4) directly attached to the tower (32) in a removable and sliding manner in a direction parallel to the longitudinal guide (21), the bar (4) being located at a distance from the frame (2) suitable for insertion into the vagina of a patient lying supine with legs bent on the radiotherapy immobiliser during radiotherapy treatment or medical imaging,
at least the bar (4) being made of a material with an electron density essentially equal to that of water.

2. Localisation device (1) according to claim 1, wherein the frame (2) and the trolley (3) are also made of a material with an electron density essentially equal to that of water.

3. Localisation device (1) according to any of the preceding claims, wherein the frame (2) comprises a square or rectangular frame (22) with the guide (21) arranged between two opposite sides and perpendicular thereto.

4. Localisation device (1) according to any of the preceding claims, wherein the guide (21) further comprises locating marks (21m) visible by means of computed tomography and magnetic resonance apparatus.

5. Localisation device (1) according to any of the preceding claims, wherein the base (31) comprises a prismatic cavity with an axis perpendicular to the plane of the frame (2), inside which a portion of the tower (32) opposite to that to which the bar (4) is attached fits.

6. Localisation device (1) according to any of the preceding claims, which further comprises an auxiliary bar (5) attached to the trolley (3) in a removable manner parallel to the bar (4).

7. Localisation device (1) according to any of the preceding claims, wherein the bars (4, 5) comprise two longitudinal slots (41, 51) located on diametrically opposite sides that are configured to slide into protrusions of a cavity in the trolley (3).

8. Localisation device (1) according to any of the preceding claims, wherein the bars (4, 5) comprise locating marks (4m, 5m) visible by means of computed tomography and magnetic resonance apparatus.

9. Localisation device (1) according to claim 8, wherein the locating marks (4m, 5m) comprise equispaced holes along the longitudinal direction of the bars (4, 5).

10. Localisation device (1) according to any of the preceding claims, which further comprises a protrusion (6) arranged in a proximal portion of the frame (2) for supporting an immobilisation mattress.

11. Localisation device (1) according to any of the preceding claims, wherein the distance between the bar (4) and the frame (2) is between 10 cm and 25 cm.

12. A localisation system (1) for radiotherapy in gynaecological tumours, **characterised in that** it comprises:
- a localisation device (1) according to any of the preceding claims;
- a plurality of replacement towers (32) for attaching to the base (32) of the trolley (3), where each tower (32) is configured for attaching the bar (4) at a different distance from the frame (2); and
- a plurality of replacement bars (4) of different diameters configured for attaching to the towers (32).
